# EUROPEAN PATENT APPLICATION

(11) **EP 4 613 840 A1**
(43) Date of publication of application: **10.09.2025**
(21) Application number: 24161621.8
(22) Date of filing: 05.03.2024
(51) Int. Cl.: C12M 1/22, C12M 1/34, G01N 33/46, G02B 21/30

(54) **MICROSCOPE TEMPERATURE STAGE**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a microscope stage (100) for temperature-controlled live cell imaging comprising a cooling device (110), a heat transfer plate (1) having a cylinder (131) open on both ends for accommodating a Petri dish (3) and a heat sink (2) configured as support (120), wherein the cylinder (131) protrudes into a circularly shaped opening of the support (120). In a preferred embodiment, the microscope stage (100) comprises an objective heater (40) for preventing heat loss due to the microscope objective.

## Description

The present invention relates to a microscope stage (100) for temperature-controlled live cell imaging comprising a cooling device (110), a heat transfer plate (1) having a cylinder (131) open on both ends for accommodating a Petri dish (3) and a heat sink (2) configured as support (120), wherein the cylinder (131) protrudes into a circularly shaped opening of the support (120). In a preferred embodiment, the microscope stage (100) comprises an objective heater (40) for preventing heat loss due to the microscope objective.

### Background of the invention

Fluorescence microscopy is a powerful tool for studying biological systems, particularly cells and (micro)organisms. The microscope stage is not the natural environment of a biological system, thereby causing death after a short period of time. Further, the relevance and robustness of the obtained results from an organism/organ/cell culture in a non physiological environment will always remain questionable. The way to improve it is to recreate the optimum sample environment (temperature, pH, nutrient supply...) on the microscope stage.

Temperature is one of the key parameters that live-organisms depend on to keep their homeostasis at each scale (organism, cells, molecules). Therefore, while performing biological experiments, temperature control is required to reduce the variability between experiments, and also to guarantee the relevance of the observed results. The temperature is a very important parameter since it can affect various biological processes, including the complete organism phenotype (e.g. sex of different species such as fish or reptiles is thermo-dependent), the cell cycle between 0 °C and 22°C is reversed in some mammalian cells (e.g. CFPAC-1 cells). Other organism such as *Caenorhabditis elegans* can suffer temporal paralysis due to drastic temperature changes.

International patent application WO 2019/101964 discloses a microscope comprising a microscope stage comprising a cooling device and a heat management system which is adapted to remove heat from the cooling element for controlling the temperature of small samples on a microscope coverslip.

The objective of the present invention is the provision of an improved microscope stage suitable for temperature-controlled imaging of larger samples, including live cells and embryos, which require air exchange.

The objective of the present invention is solved by the teaching of the independent claims. Further advantageous features, aspects and details of the invention are evident from the dependent claims, the description, the figures, and the examples of the present application.

### Brief description of the invention

The objective has been solved by a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

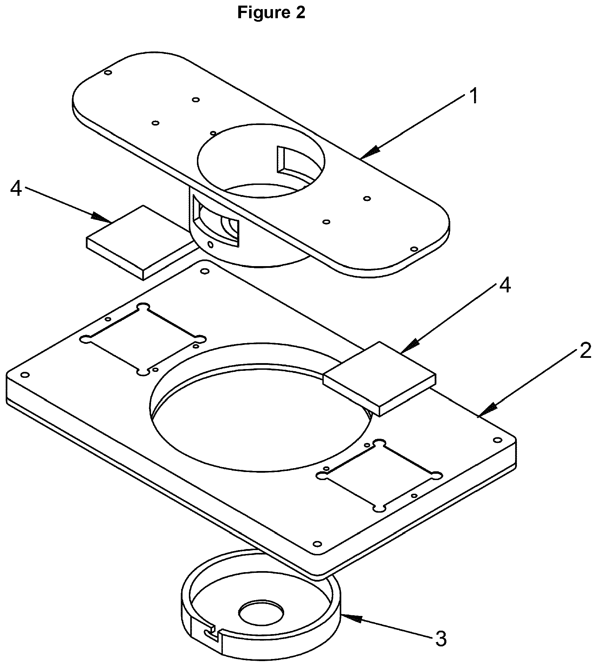

Thus, the microscope stage (100) according to the present invention comprises a cooling device (110) sandwiched between a heat transfer plate (1) and a heat sink (2) (see Figure 2), wherein the heat sink (2) removes the excess heat from the cooling device (110) or spends heat to the cooling device (110) depending on the mode of operation of the cooling device (110), i.e. whether it is configured to cool the heat transfer plate (1) or to heat the heat transfer plate (1).

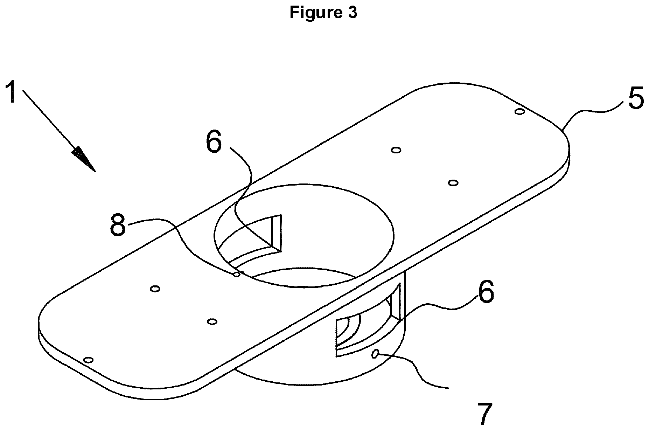

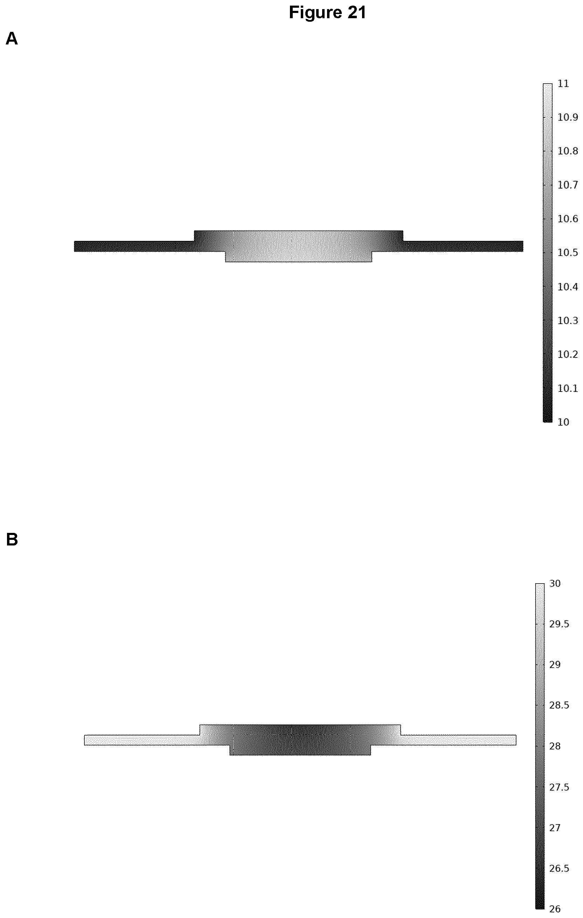

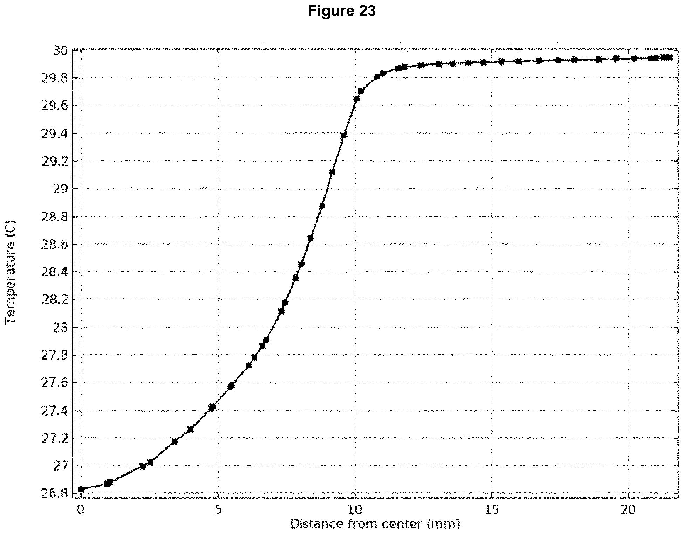

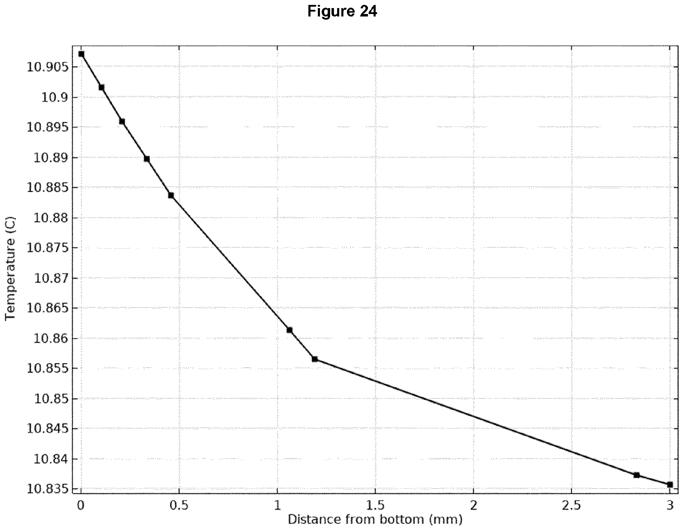

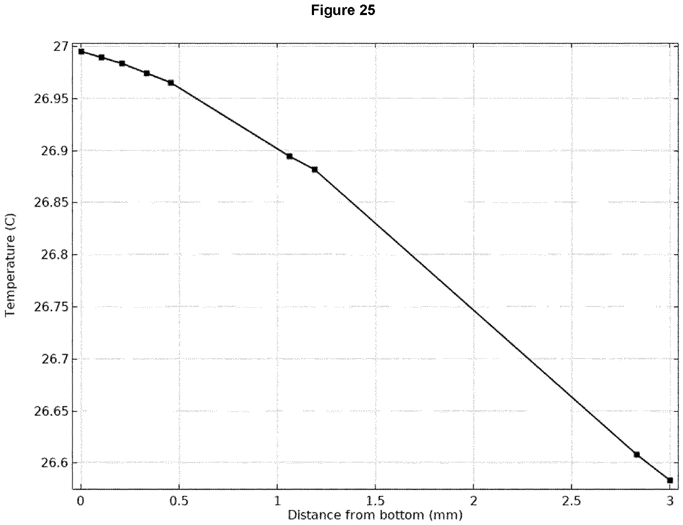

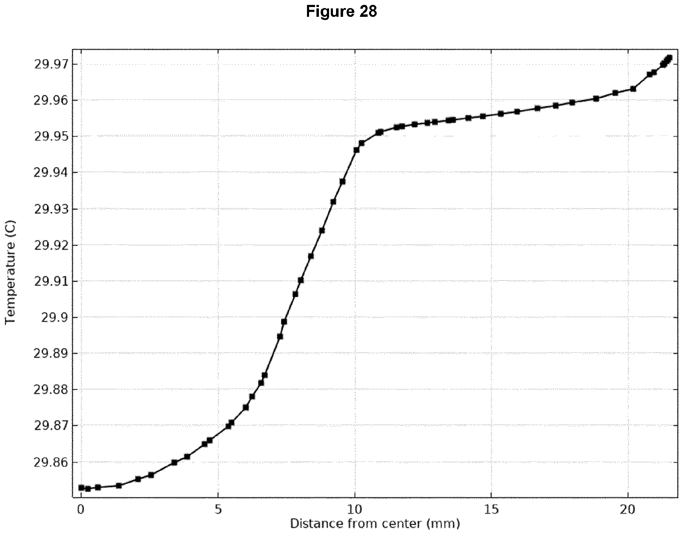

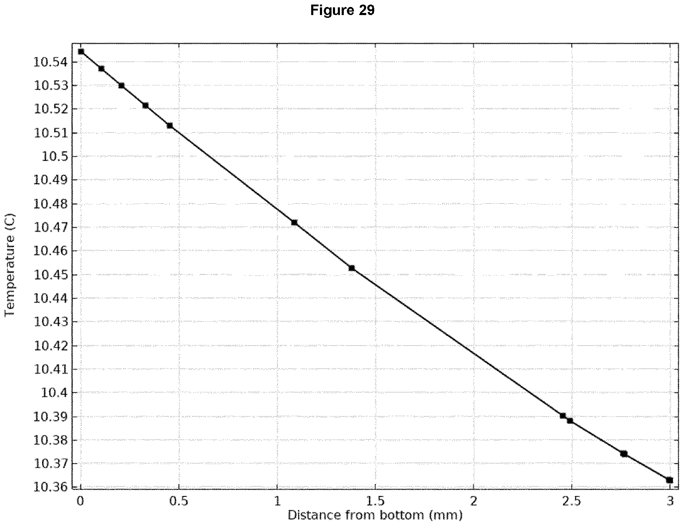

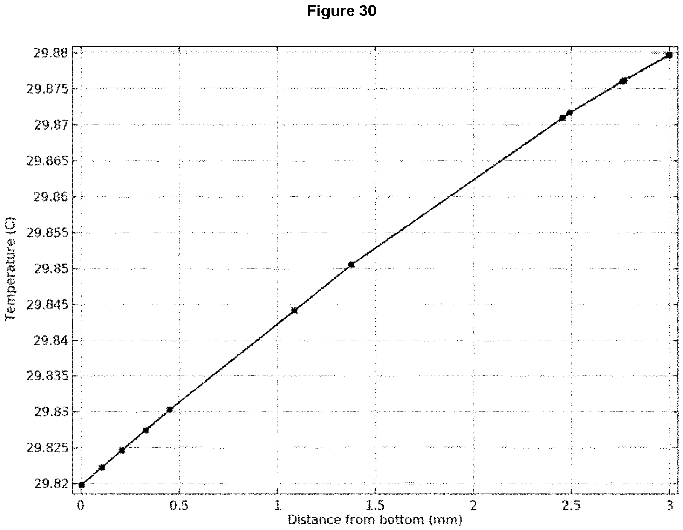

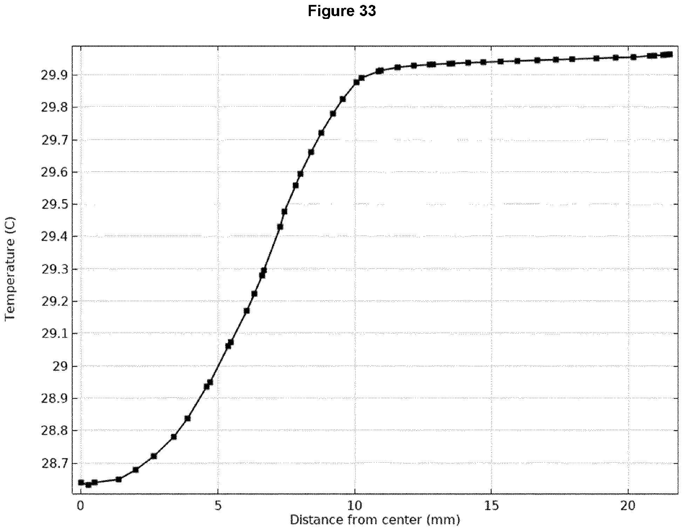

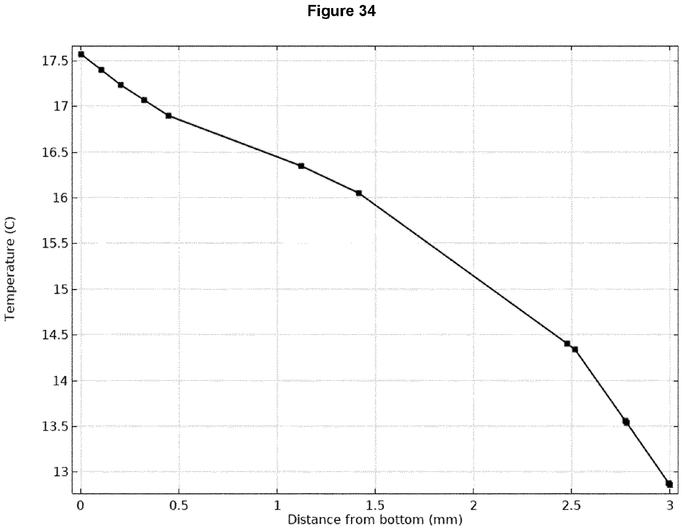

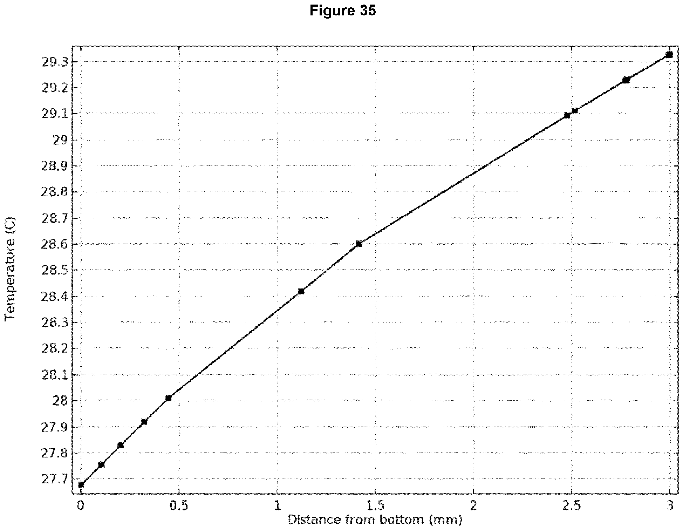

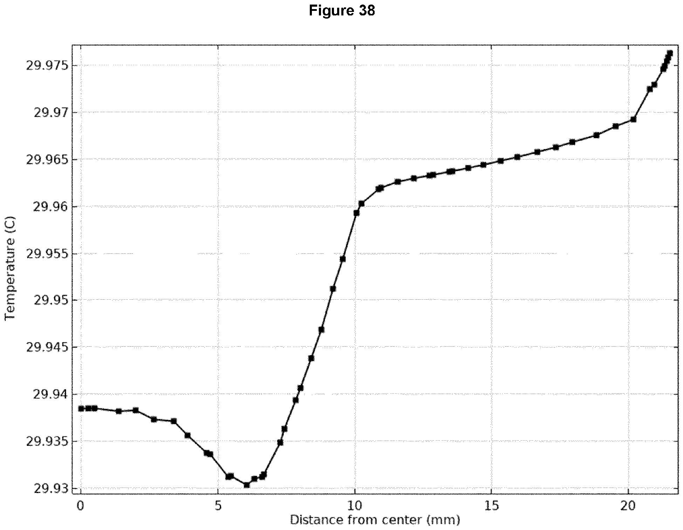

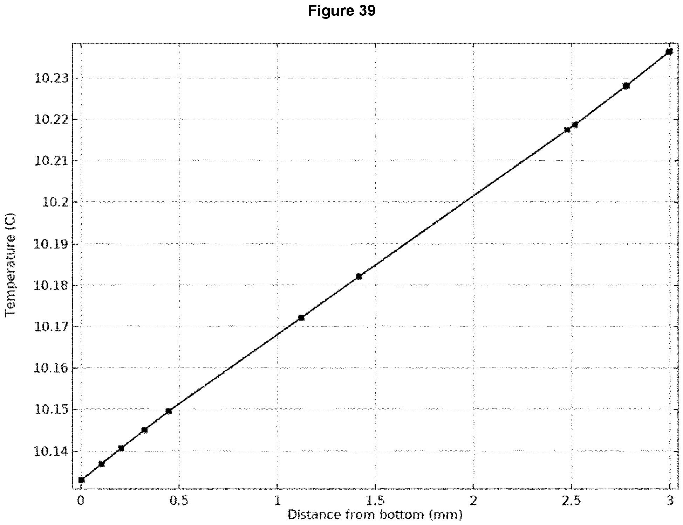

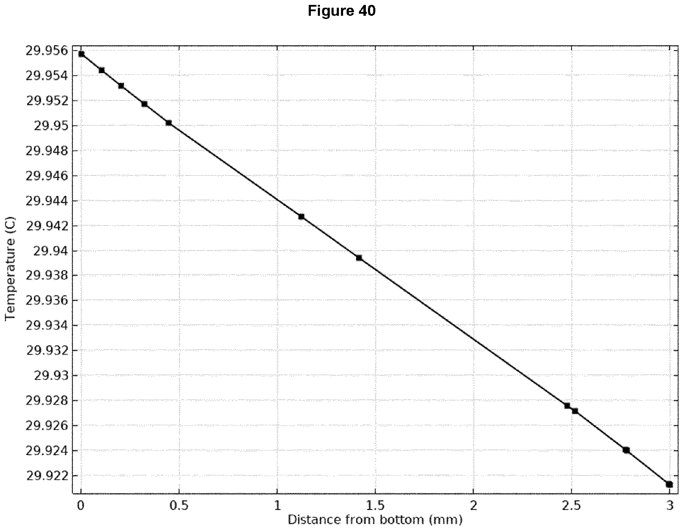

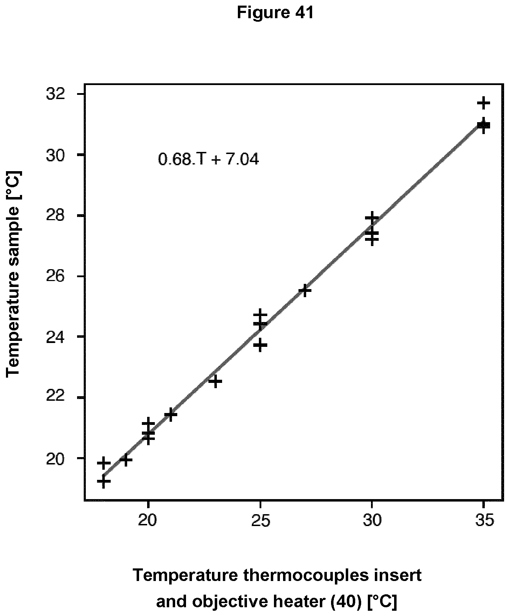

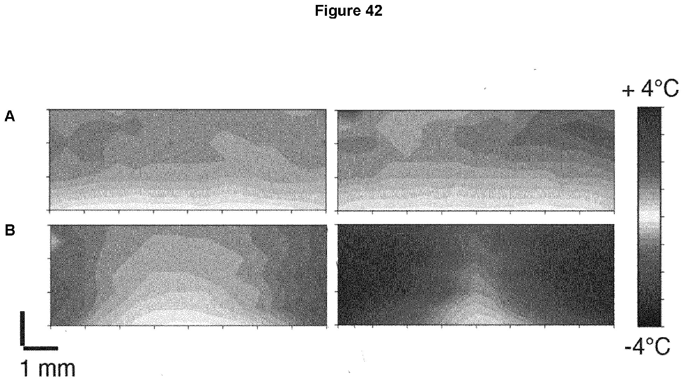

The heat transfer plate (1) of the microscope stage (100) according to the present invention comprises a cylinder open on both ends (131), which extends from the plate (1) and is adapted for receiving opposite to the plate (1) a Petri dish (3) containing a live cell sample (136) in thermal communication (see Figure 3). The cylinder of the plate (1) holds the Petri dish (3) while simultaneously transfers heat to the sample in the Petri dish (3). Plate (1) and Petri dish (3) are in thermal communication to enable efficient temperature control of the sample. Thus, this inventive configuration allows efficient and precise temperature control of a biological sample with a thickness larger than 100 µm in a Petri dish format (see figures 21 to 42). Due to the open cylinder configuration oxygen exchange (via) of the sample is possible.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein an insert (10) made of sapphire is placed between the opening of the cylinder (131) opposite to the plate (1) and the Petri dish (3) to reduce the sample volume and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the cylinder (131) comprises a pin for holding the Petri dish (3) in position, wherein an insert (10) made of sapphire is placed between the opening of the cylinder (131) opposite to the plate (1) and the Petri dish (3) to reduce the sample volume and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), wherein the cylinder (131) comprises a pin for holding the Petri dish (3) in position, wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) made of heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

Another aspect of the present invention is directed to a microscope (1000) comprising a microscope stage (100) as disclosed herein.

### Description of the invention

The term "heater" as used herein, such as in "objective heater", refers to a device capable increasing (heating) or decreasing (cooling) the temperature of an object, such as an objective or a sample. Thus, a "cooling device" is also a "heating device", a "cooling element" is a "heating element", and a "cooler" is also a "heater".

The "cooling liquid" is generally a substance comprising a chemical element or a chemical compound or composition which can be provided in a liquid state in the temperature ranges of interest for cooling or heating the sample. Preferably, the cooling liquid is water

"Room temperature", as used herein, refers to temperatures greater than 4 °C, preferably from 15-40 °C, 15 °C to 30 °C, and 15 °C to 24 °C, and 16 °C to 21 °C. Such temperatures include 14 °C, 15 °C, 16 °C, 17 °C, 18 °C, 19 °C, 20 °C, and 21 °C.

"Inverse microscopy" as used herein, refers to any type microscopy in which the objectives of the microscope are located under the stage, pointing up and thereby observing the sample from below. Therefore, inverse microscopy includes, but is not restricted to, confocal microscopy, (multi-modal) epifluorescence microscopy, wide-field fluorescence microscopy, super-resolution microscopy, like single molecule localization microscopy, stimulated emission depletion microscopy (STED), fluorescence-lifetime imaging microscopy with time-correlated single-photon counting, electron microscopy.

"Sample", as used herein, refers to any specimen, which can be analyzed in a Petri dish by a microscope by using the inventive microscope stage (100). In general, each kind of sample can be cooled down. The sample generally comprises a variable liquid composition of substances, such as soft matter, materials, chemical reaction partners or particularly preferred biological materials. Preferably, the sample comprises living biological material and more preferably living cells, living biological cells, living cell components or organoids. The cells form an adherent cell culture, preferably in the form of a cell monolayer, which can have a thickness of 10 µm. The sample may also have a thickness of at least 80 µm, more preferably of at least 90 µm, more preferably of at least 95 µm, and most preferably of at least 100 µm.

### Heat transfer plate (1)

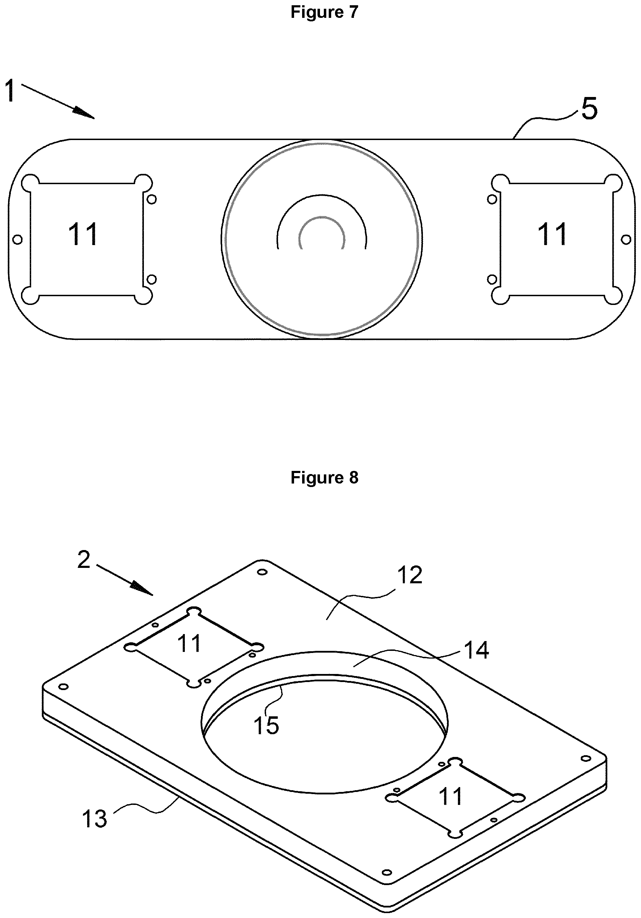

The heat transfer plate (1) of the inventive microscope stage (100) conducts heat from the cooling device (110) to the sample in the Petri dish (3) and accommodates the Petri dish (3) in the optical path of the microscope. To this extent, the heat transfer plate (1) of the inventive microscope stage (100) has the shape of an elongated plate with a circular opening in the center. A cylinder (131) extends from the circular opening of the plate (1) and is open on both ends. The cylinder comprises an opening (6) in its wall to allow sample observation, facilitating proper sample positioning (see Figures 3 to 8). Thus, heat is conducted to the Petri dish via the cylinder (131). The cylinder (131) further comprises a channel (8) in its wall configured to accommodate a thermocouple (141) at the end of the cylinder opposite to the plate (1). The thermocouple (141) may be a type K thermocouple or an NTC thermocouple.

The heat transfer plate (1) of the inventive microscope stage (100) is made of a heat conductive material, preferably aluminium or copper.

The cylinder (131) further comprises a locking means (7) for receiving opposite to the plate (1) a Petri dish (3) containing a live cell sample (136) in thermal communication. Said locking mean (7) is preferably at least one pin or at least one screw, preferably one pin. The skilled person readily envisions that the Petri dish is modified with a thread, opening, hole, recess or cut out (21) compatible with the locking means (7).

In one embodiment of the inventive microscope stage (100), the opening of the cylinder (131) opposite to the plate is covered from inside the cylinder with a transparent coverslip (9) made of sapphire or glass to close the opening of the cylinder after mounting the Petri dish (3).

In one embodiment of the inventive microscope stage (100), the opening of the cylinder (131) opposite to the plate is reduced with an insert (10), preferably made of a heat conductive and transparent material such as sapphire to maintain visualization and heat conduction. Glass is not a preferred material for the insert (10). Insert (10) improves the heat distribution within the sample and facilitates cleaning of the stage since the insert (10) is removable and the sample does not get into contact with the material of the heat transfer plate (1).

The sample volume is limited by the transparent coverslip (9) and the insert (10). Thus, the volume of the sample chamber can be increased by removing the insert (10) or lengthening the cylinder border or by changing the height of the locking means (7).

The cylinder (131) is preferably adapted to receive and hold the Petri dish (3) such that the Petri dish (3) does not contact the heat sink (2). Further, the cylinder (131) is preferably adapted to receive and hold the Petri dish (3) such that the Petri dish (3) does not cover the opening (6).

In a preferred embodiment, the heat transfer plate (1) further comprises thin cut outs (11a) or depressions on the side on which the cylinder (131) extends to receive the at least one cooling element (4) of the cooling device (110). Thus, the thin cut outs (11a) or depressions are in the shape of the at least one cooling element (4).

Thus, one embodiment is directed to a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the heat transfer plate (1) further comprises thin cut outs (11a) or depressions on the side on which the cylinder (131) extends to receive the at least one cooling element (4) of the cooling device (110).

The cylinder (131) is preferably longer than the height of the heat sink (2), thereby the Petri dish (3) can be attached on the side of the heat sink (2) opposite to the heat transfer plate (1).

The opening (6) is preferably positioned on the wall of the cylinder (131) so that the opening (6) is visible between the Petri dish (3) when mounted and the heat sink (2)

### Heat sink (2)

The heat sink (2) of the inventive microscope stage (100) absorbs excessive heat produced by the at least one cooling element (4) or conducts heat to the at least one cooling element (4) depending on the mode of operation of the at least one cooling element (4). The heat sink (2) is in thermal communication with the cooling element.

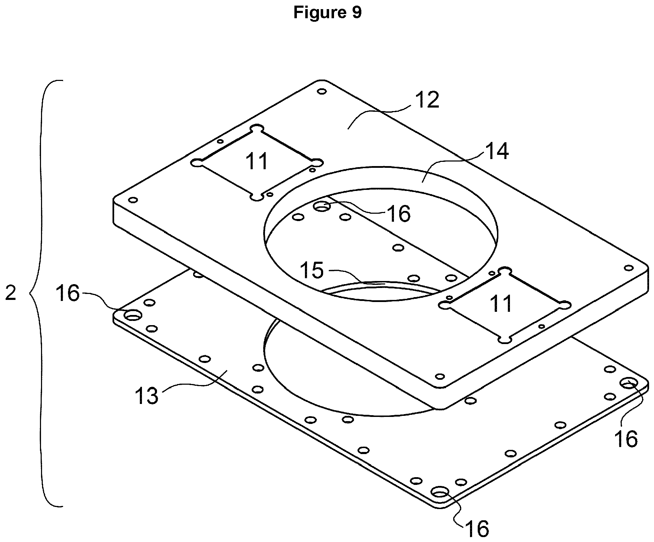

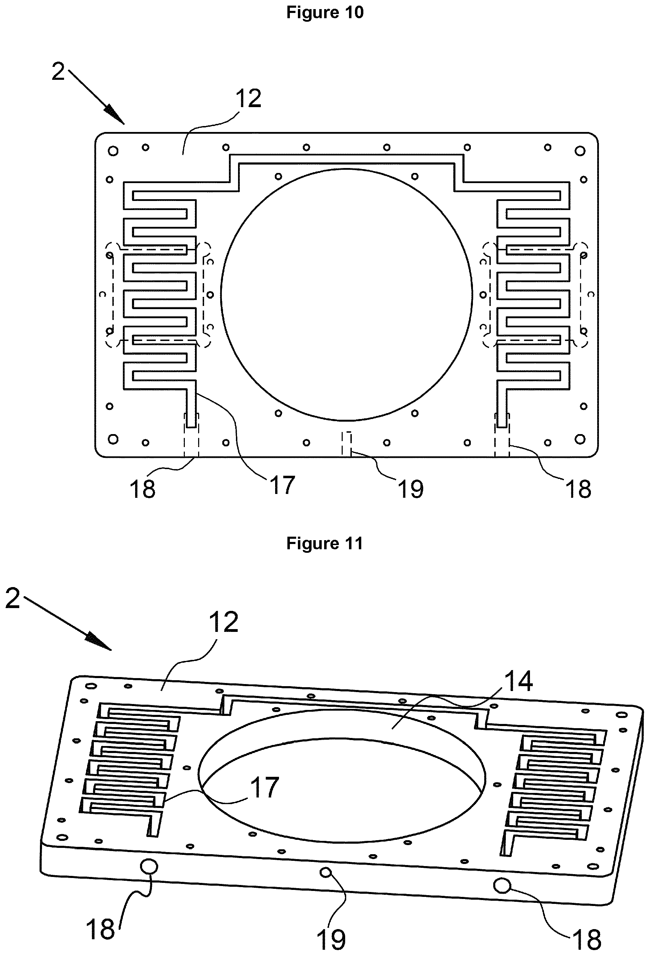

The heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142) (see Figures 9 to 11). The thermocouple (142) may be a type K thermocouple or an NTC thermocouple.

The cooling liquid channel (17) forms a pathway from the inlet to the outlet around the circular opening (14), thereby allowing efficient and uniform heat absorption over the whole heat sink (2).

For an easier supply and removal of the cooling liquid, the inlet and outlet (18) are located at the same side of the heat sink. The cooling liquid is pumped by a pump from the inlet through the cooling liquid channel (17) to the outlet.

The heat sink (2), heat transfer plate (1) and the cooling device (110) can be held in place by using screws.

Thus, one embodiment is directed to a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cooling liquid channel (17) forms a pathway from the inlet to the outlet around the circular opening (14), thereby allowing efficient and uniform heat absorption over the whole heat sink (2).

Since the heat sink (2) is configured as a support (120) for the heat transfer plate (1), the cooling device (110) and the Petri dish (3) (via cylinder 131) the heat sink is made of a stable and heat conductive material, preferably aluminium or copper. Thus, the top plate (12) and the lid (13) are preferably made of aluminium.

In a preferred embodiment, the outer side of the top plate (12) of the heat sink (2) further comprises thin cut outs (11b) or depressions to receive the at least one cooling element (4) of the cooling device (110). Thus, the thin cut outs (11b) or depressions are in the shape of the at least one cooling element (4).

Thus, one embodiment is directed to a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and the outer side of the top plate (12) of the heat sink (2) further comprises thin cut outs (11b) or depressions to receive the at least one cooling element (4) of the cooling device (110).

The thin cut outs (11a) or depressions as well as the thin cut outs (11b) or depressions improve the thermal connection between the at least one cooling element (4), the heat sink (2) and the heat transfer plate (1).

Thus, one embodiment is directed to a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), the outer side of the top plate (12) of the heat sink (2) further comprises thin cut outs (11b) or depressions to receive the at least one cooling element (4) of the cooling device (110), and wherein the heat transfer plate (1) further comprises thin cut outs (11a) or depressions on the side on which the cylinder (131) extends to receive the at least one cooling element (4) of the cooling device (110).

### Cooling device (110)

The cooling device (110) of the inventive microscope stage (100) comprises at least one cooling element (4) positioned between the heat sink (2) and the heat transfer plate (1). The at least one cooling element (4) is in thermal contact with the heat sink (2) and the heat transfer plate (1). The person skilled in the art understands that every cooling element also outputs heat, particularly in the form of waste heat that needs to be dissipated. Therefore, the heat sink (2) is adapted to remove heat from the at least one cooling element (4), wherein the heat transfer plate (1), which is in contact with the cooling element on the opposite side, is cooled by the cooling element (4).

Thermal connection between the at least one cooling element (4), heat sink (2) and the heat transfer plate (1) can be further improved by using thermal paste or thermal pads.

As already mentioned herein, the cooling element (4) may be used inverse, so that the heat transfer plate (1) is heated and the heat sink (2) is cooled down, i.e. outputs heat to the cooling element (4). Thus, the inventive microscope stage (100) can be used for heating and/or cooling a sample in the Petri dish (3).

In a preferred embodiment, at least one cooling element (4) is a thermoelectric element or Peltier element. More preferably, the at least one cooling element (4) are two Peltier elements (112,113).. More preferably, the at least one cooling element (4) are two Peltier elements (112,113) located opposite the circular opening of the heat sink (2).

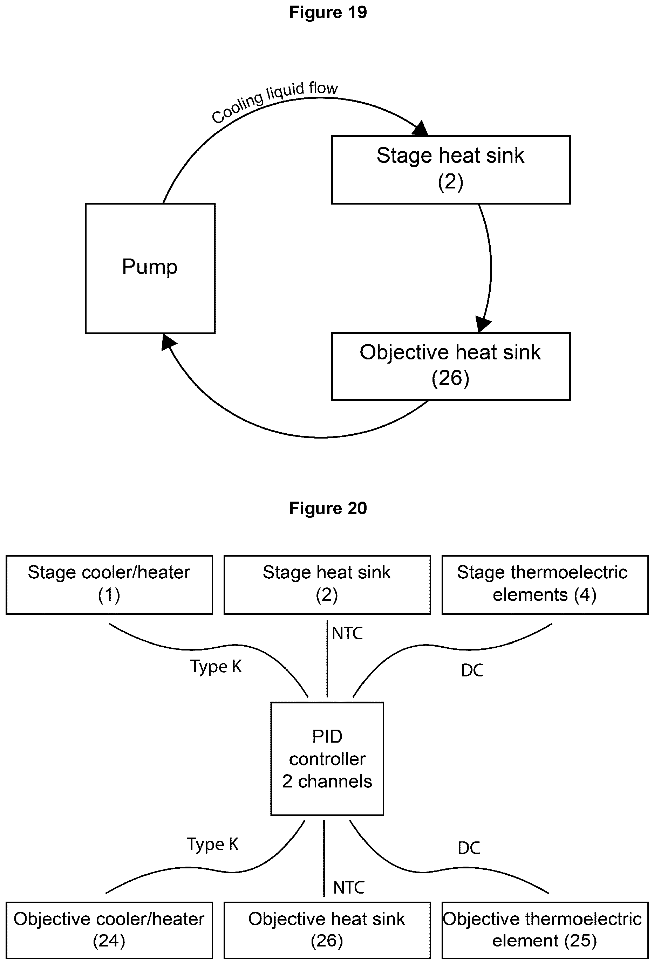

The at least one cooling elements (4) may be controlled by a PID controller (114). The PID controller (114) is connected to a computer (150) on which a software is running that allows to set a target temperature, to program temperature profiles over time and to record and export the temperature profiles during the course of an experiment. The PID controller (114) may also be used to monitor the thermocouples (141,143) (see Figure 20).

### Objective heater (40)

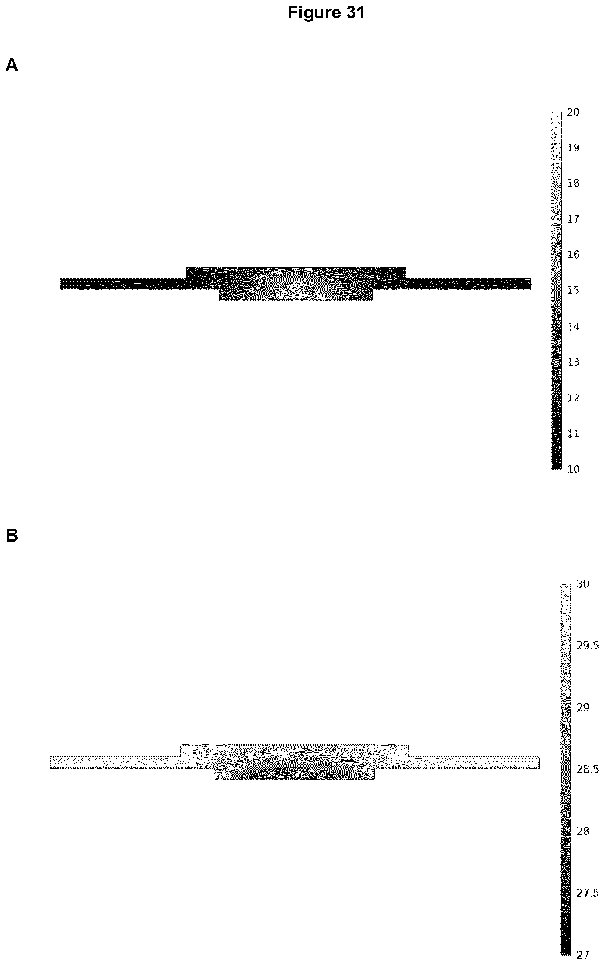

The inventors have observed that the use of immersion objectives (with an immersion oil) created a significant temperature gradient inside the Petri dish sample, as the contact with objective causes heat transfer (see Figure 31).

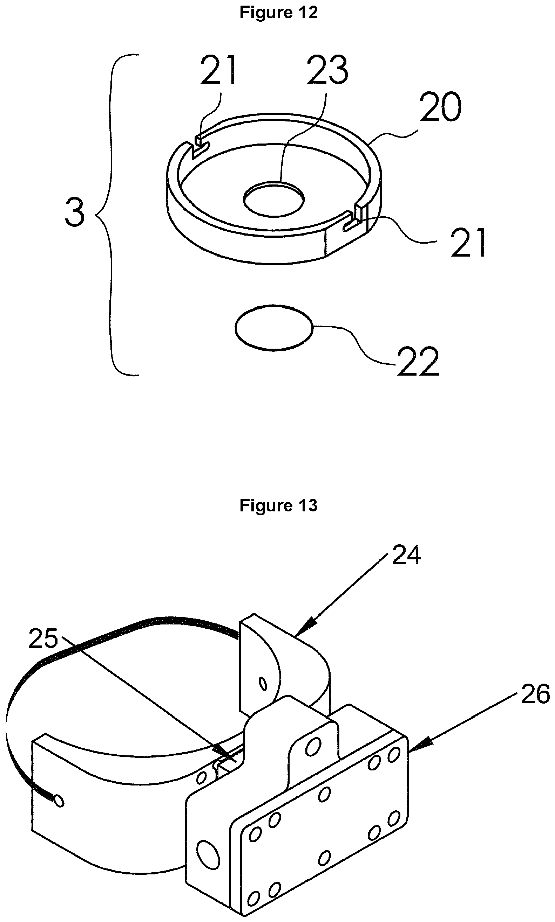

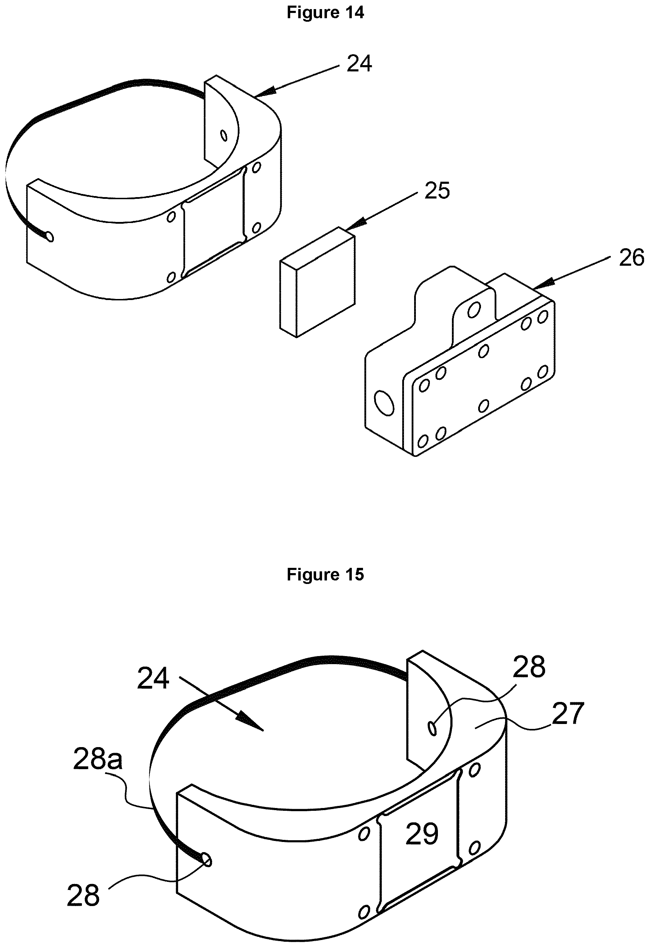

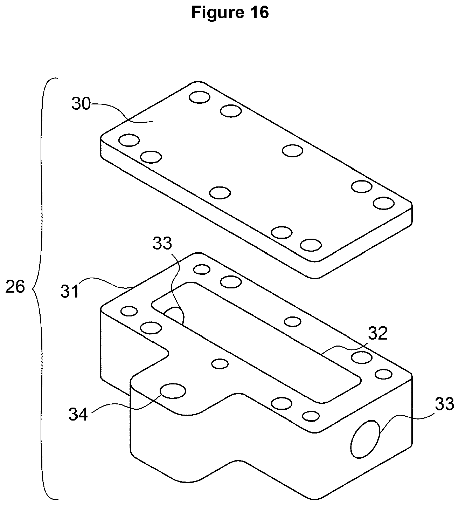

In order to minimize the heat transfer to the microscope objective, the inventive microscope stage (100) may further comprise an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25) (see Figures 13 to 16).

Thus, the objective cooler (40) comprises a heat conductive element (24) being in contact with the objective, a cooling element (25) to control the temperature of the objective (by heating or cooling), and a liquid cooling heat sink (26) to dissipate the heat generated by the cooling element (25).

The cooling element (25) may be a thermoelectric element or a Peltier element.

The cooling element (25) is in thermal contact with the heat conductive element (24) and the liquid cooling heat sink (26). Preferably, the cooling element (25) is positioned between the heat conductive element (24) and the liquid cooling heat sink (26).

In a preferred embodiment, the heat conductive element (24) comprises a body (27) preferably made of aluminium with an opening matching the circumference of the objective, holding means (28) (pin or screw) to hold an elastic band (28a) that secures the objective heater on the objective, and a thin cut out (29) or depression to help position the cooling element.

In a preferred embodiment, the liquid cooling heat sink (26) comprises a heat sink lid (30), a heat sink body (31), a channel (32) for liquid cooling, an inlet and outlet (33) for cooling liquid, and a hole (34) for positioning a thermocouple to measure the temperature of the heat sink. The thermocouple (143) is preferably a type K thermocouple or an NTC thermocouple.

As shown in Figure 19, the he cooling liquid is pumped by a pump in a closed loop first through the heat sink (2) and then through the liquid cooling heat sink (26) back to the pump. In an alternative embodiment, the cooling liquid is pumped in the reverse direction, first through the liquid cooling heat sink (26) and then through the heat sink (2) back to the pump (not shown in Figure 19).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and
wherein the liquid cooling heat sink (26) comprises a lid (30) and a body (31),
wherein the body (31) comprises a channel (32) for liquid cooling connected to an inlet and outlet (33), and an opening (34) configured to accommodate a thermocouple (143) for measuring the temperature of the liquid cooling heat sink (26).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein an insert (10) made of sapphire is placed between the opening of the cylinder (131) opposite to the plate (1) and the Petri dish (3) to reduce the sample volume and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the cylinder (131) comprises a pin for holding the Petri dish (3) in position, wherein an insert (10) made of sapphire is placed between the opening of the cylinder (131) opposite to the plate (1) and the Petri dish (3) to reduce the sample volume and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), wherein the cylinder (131) comprises a pin for holding the Petri dish (3) in position, wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122), and
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

### Spacer (36)

The inventors have found that the objective heater (40) works more efficient when a spacer (36) is placed between the objective and the microscope body, thereby thermally isolating the objective from the microscope body. Therefore, the spacer is made of a thermally non conductive material, preferably plastic.

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the liquid cooling heat sink (26) comprises a lid (30) and a body (31), wherein the body (31) comprises a channel (32) for liquid cooling connected to an inlet and outlet (33), and an opening (34) configured to accommodate a thermocouple (143) for measuring the temperature of the liquid cooling heat sink (26).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume.

In a preferred embodiment, the microscope stage (100) for temperature-controlled live cell imaging comprises:
(a) a cooling device (110) comprising at least one cooling element (4);
(b) a heat transfer plate (1) made of a heat conductive material, preferably aluminium comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1);
(c) a heat sink (2) made of a heat conductive material, preferably aluminium configured as support (120) comprising a circularly shaped opening (122),
(d) an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and
(e) a spacer (36) for thermal isolation positioned between the objective and the microscope body,

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the at least one cooling element (4) are two Peltier elements (112,113) controlled by a PID controller (114) connected to a computer (150), wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142), and wherein the cylinder (131) comprises a pin for holding the Petri dish (3) in position, and wherein an insert (10) made of sapphire is placed between the opening of the cylinder (131) opposite to the plate (1) and the Petri dish (3) to reduce the sample volume, and wherein the opening of the cylinder (131) opposite to the plate is covered from inside the cylinder with a transparent coverslip (9) made of sapphire or glass.

### Microscope (1000)

Further advantages of the invention result from the temperature control of the sample in the Petri dish (3). For example, the inventive microscope stage (100) allows the arrangement of a microscope (1000) for optical microscopy of the sample in the Petri dish (3) while controlling the temperature of the sample efficiently. The microscope (1000) may be any suitable microscope, for example, an optical microscope, a confocal microscope, an inverse confocal microscope, or an observation optical system and/or an impedance measuring device, especially an inverse optical microscope.

Thus, another aspect of the present invention is directed to a microscope (1000) comprising a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the opening of the cylinder (131) opposite to the plate is covered from inside the cylinder with a transparent coverslip (9) made of sapphire or glass.

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein an insert (10) made of sapphire is placed between the opening of the cylinder (131) opposite to the plate (1) and the Petri dish (3) to reduce the sample volume.

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the cylinder (131) comprises a pin for holding the Petri dish (3) in position.

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the stage further comprises an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25).

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the stage further comprises an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25) wherein the liquid cooling heat sink (26) comprises a lid (30) and a body (31), wherein the body (31) comprises a channel (32) for liquid cooling connected to an inlet and outlet (33), and an opening (34) configured to accommodate a thermocouple (143) for measuring the temperature of the liquid cooling heat sink (26).

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), wherein the stage further comprises an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25), and wherein the stage (100) further comprises a spacer (36) for thermal isolation positioned between the objective and the microscope body.

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113).

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the at least one cooling element (4) are two Peltier elements (112,113) controlled by a PID controller (114) connected to a computer (150).

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the heat transfer plate (1) is made of a heat conductive material, preferably aluminium.

In a preferred embodiment, the microscope (1000) comprises a microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),

wherein the cooling device (110) is mounted on the support (120) to enable the heat sink (2) to dissipate heat produced by the at least one cooling element (4),
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136), and wherein the heat sink (2) is made of a heat conductive material, preferably aluminium.

### Description of the Figures

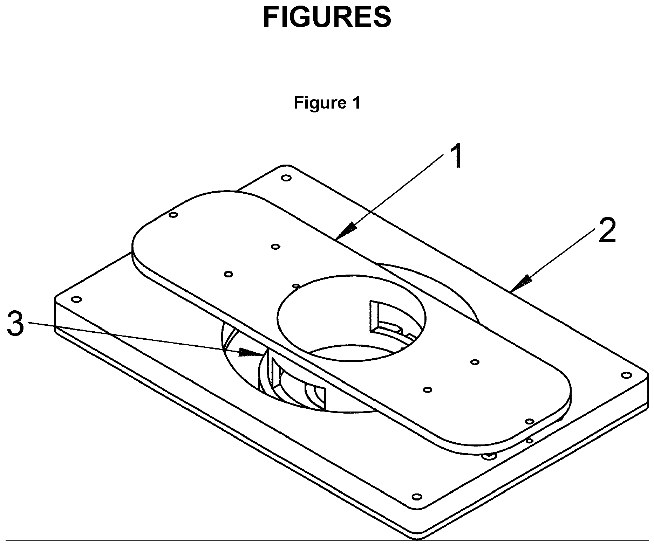

- **Fig. 1**: shows a schematic view of three parts of the four parts of the inventive microscope stage. Heat transfer plate (1), heat sink (2) connected to a water flow to keep the temperature constant on the other side of the cooling elements, and modified Petri dish to fit in the cylinder of 1.
- **Fig. 2**: shows an exploded view of an exemplary embodiment of the inventive microscope stage. Depicted are: heat transfer plate (1), which conducts heat from the cooling elements (4) to the cylinder where the Petri dish (3) is mounted, heat sink (2) with liquid cooling system to dissipate the heat from the opposite side of the cooling elements. Modified Petri dish (3) that fits into the cylinder of 1. cooling elements (4) to control the temperature of the Petri dish. Thermal connections between the cooling elements (4) and 1 and 2 can be achieved either by thermal paste or by thermal pads. The system can be held in place by using screws.
- **Fig. 3**: shows an isometric view of an exemplary embodiment of the heat transfer plate (1) preferably made of aluminium: Body (5) of 1 having a cylinder that conducts heat to the sample in the Petri dish, opening (6) in the frame to permit sample visualization, screw or pin (7) to lock the Petri dish in position, and channel (8) to insert the type K thermocouple.

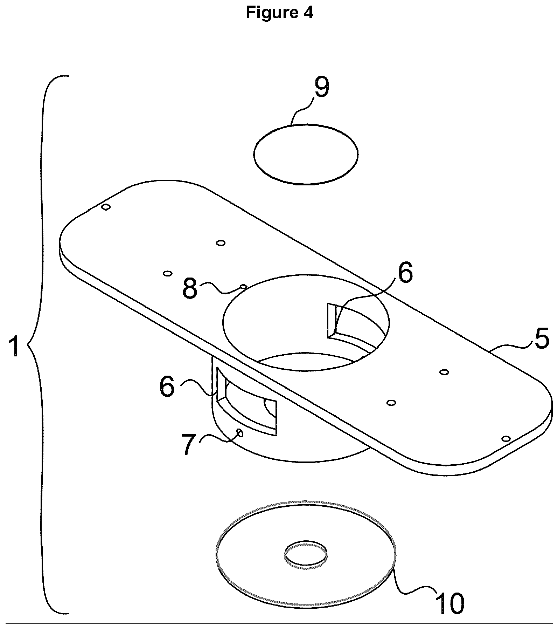

- **Fig. 4**: shows an exploded view of an exemplary embodiment of the heat transfer plate (1) preferably made of aluminium: Body (5) of 1 having a cylinder that conducts heat to the sample in the Petri dish, opening (6) in the frame to permit sample visualization, locking means (screw or pin) (7) to lock the Petri dish in position, channel (8) to insert the type K thermocouple, coverslip (9), preferably made of sapphire or glass, to close the opening of the cylinder after mounting the sample, and optional insert (10) made of sapphire to reduce the opening of the cylinder while permitting visualization and heat conduction.
- **Fig. 5**: shows an exploded view of an exemplary embodiment of the heat transfer plate (1) preferably made of aluminium: Body (5) of 1 having a cylinder that conducts heat to the sample in the Petri dish, channel (8) to insert the type K thermocouple, coverslip (9), preferably made of sapphire or glass, to close the opening of the cylinder after mounting the sample, optional sapphire insert (10) to reduce the opening of the cylinder while permitting visualization and heat conduction. Dashed line indicates the cutting plane for cross-section in Fig. 6.

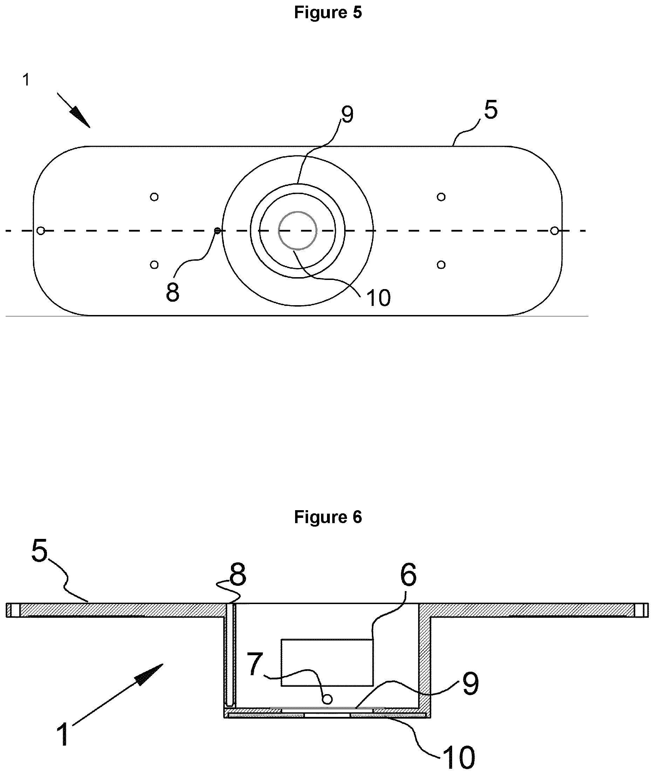

- **Fig. 6**: shows the longitudinal section view of an exemplary embodiment of the heat transfer plate (1) preferably made of aluminium: Body (5) of 1 having a cylinder that conducts heat to the sample in the Petri dish, opening (6) in the frame to permit sample visualization, locking means (7) (preferably screw or pin) to lock the Petri dish in position, channel (8) to insert the type K thermocouple, coverslip (9), preferably made of sapphire or glass, to close the opening of the cylinder after mounting the sample, optional sapphire insert (10) to reduce the opening of the cylinder while permitting visualization and heat conduction. The sample volume is represented by the white space between 9 and 10. The sample chamber can be increased by removing 10 or lengthening the cylinder border or by changing the height of 7. The thermocouple is located at the end of the channel on the bottom plate of the cylinder.
- **Fig. 7**: shows a bottom view of an exemplary embodiment of the heat transfer plate (1) preferably made of aluminium: body (5) of 1 having a cylinder that conducts heat to the sample in the Petri dish. Thin cut outs (11a) or depressions help to position the cooling elements.
- **Fig. 8**: shows an isometric view of an exemplary embodiment of the heat sink (2): Thin cut outs (11b) or depressions that help to position the cooling elements, top plate (12) of heat sink (2) including channels for liquid cooling (not shown), lid (13) of heat sink (2), opening (14) in 12 where the cylinder goes through, opening (15) in lid (13) where the cylinder of 1 goes through.
- **Fig. 9**: shows an exploded view of an exemplary embodiment of the heat sink (2): thin cut outs (11b) or depressions that help to position the cooling elements, top plate (12) of 2 including channels for liquid cooling, lid (13) of the heat sink (2), opening (14) in 12 where the cylinder goes through, opening (15) in lid (13) where the cylinder of 1 goes through, and holes (16) used for positioning of the temperature stage in the microscope's stage adapter.
- **Fig. 10**: shows a bottom view of an exemplary embodiment of the heat sink (2): top plate (12) of the heat sink (2) including channels for liquid cooling, cooling liquid channel (17), cooling liquid inlet and outlet (18), and opening (19) for positioning an NTC thermocouple to read the temperature of the heat sink.
- **Fig. 11**: shows an isometric view of an exemplary embodiment of the heat sink (2): top plate (12) of the heat sink (2) including channels for liquid cooling, opening (14) in 12 where the cylinder goes through, cooling liquid channel (17), Cooling liquid inlet and outlet (18), and opening (19) for positioning an NTC thermocouple to read the temperature of the heat sink.
- **Fig. 12**: shows an exploded view of an exemplary embodiment of the modified Petri dish (3): Petri dish body (20), cut outs (21) for locking the Petri dish in the cylinder, and a coverslip (22), preferably made of glass to seal the opening in 20. This is the visualization area and where the sample is mounted. Opening (23) in 20 to permit imaging of the sample.
- **Fig. 13**: shows an isometric view of an exemplary embodiment of the objective heater (40) comprising a heat conductive element (24) being in contact with the objective, a cooling element (25) to control the temperature of the objective (by heating or cooling), and a liquid cooling heat sink (26) to dissipate the heat generated by 25.
- **Fig. 14**: shows an explosive view of an exemplary embodiment of the objective cooler (40) a comprising heat conductive element (24) being in contact with the objective, a cooling element (25) to control the temperature of the objective (by heating or cooling), and a liquid cooling heat sink (26) to dissipate the heat generated by 25.
- **Fig. 15**: shows an isometric view of an exemplary embodiment of the heat conductive element (24) comprising a body (27) preferably made of aluminium with an opening matching the circumference of the objective, holding means (28) (pin or screw) to hold an elastic band (28a) that secures the objective heater on the objective, and a thin cut out (29) or depression to help position the cooling element.
- **Fig. 16**: shows an isometric view of an exemplary embodiment of the a liquid cooling heat sink (26) comprising a heat sink lid (30), a heat sink body (31), a channel (32) for liquid cooling, an inlet and outlet (33) for cooling liquid, and a hole (34) for positioning a thermocouple to measure the temperature of the heat sink.

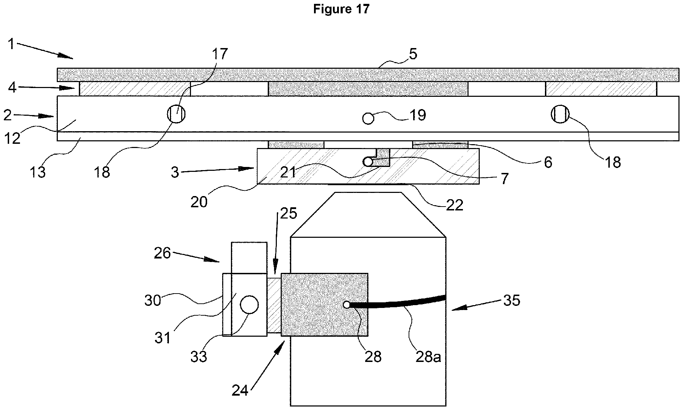

- **Fig. 17**: shows a front view of a fully assembled embodiment of the inventive microscope stage and a microscope objective (35) for reference, the stage comprising a heat transfer plate (1), a heat sink (2) connected to a cooling liquid flow to keep the temperature constant on the other side of the cooling elements, a modified Petri dish (3) to fit in the cylinder of 1, cooling elements (4) to control the temperature of the Petri dish, a body (5) of 1 having a cylinder that conducts heat to the sample in the Petri dish, an opening (6) in 5 to permit sample visualization, locking means (7) (screw or pin) to lock the Petri dish in position, a top plate (12) of 2 including channels for liquid cooling, lid (13) of heat sink (2), a cooling liquid inlet and outlet (18), an opening (19) for positioning a thermocouple to read the temperature of the heat sink, a Petri dish body (20), cut outs (21) or depressions that permit to lock 3 in the cylinder, a coverslip (22) (preferably made of glass) to seal the opening in 20 and which used for sample visualization, a heat conductive element (24) that is in contact with the objective, a cooling element (25) to control the temperature of the objective, liquid cooling heat sink (26) to dissipate the heat generated by 25, holding means (28) (pin or screw) to hold the elastic band (28a) that secures the objective heater on the objective, a heat sink lid (30) of 26, a heat sink body (31), a channel for liquid cooling (32), and an inlet and outlet (33) for cooling liquid.

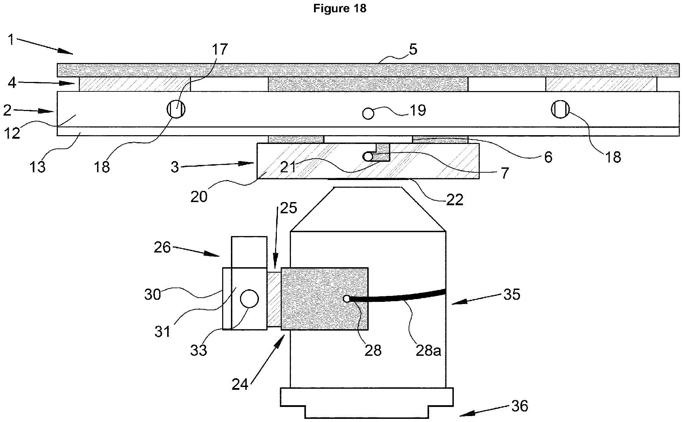

- **Fig. 18**: shows a front view of a fully assembled embodiment of the inventive microscope stage and a microscope objective (35) for reference, the stage comprising a heat transfer plate (1), a heat sink (2) connected to a cooling liquid flow to keep the temperature constant on the other side of the cooling elements, a modified Petri dish (3) to fit in the cylinder of 1, cooling elements (4) to control the temperature of the Petri dish, a body (5) of 1 having a cylinder that conducts heat to the sample in the Petri dish, an opening (6) in 5 to permit sample visualization, locking means (7) (screw or pin) to lock the Petri dish in position, a top plate (12) of 2 including channels for liquid cooling, lid (13) of heat sink (2), a cooling liquid inlet and outlet (18), an opening (19) for positioning an NTC thermocouple to read the temperature of the heat sink, a Petri dish body (20), cut outs (21) or depressions that permit to lock 3 in the cylinder, a coverslip (22) (preferably made of glass) to seal the opening in 20 and which used for sample visualization, a heat conductive element (24) that is in contact with the objective, a cooling element (25) to control the temperature of the objective, liquid cooling heat sink (26) to dissipate the heat generated by 25, holding means (28) (pin or screw) to hold the elastic band (28a) that secures the objective heater on the objective, a heat sink lid (30) of 26, a heat sink body (31), a channel for liquid cooling (32), an inlet and outlet (33) for cooling liquid, and a spacer (36) which thermally isolates the objective from the microscope body.
- **Fig. 19**: shows a scheme of the cooling liquid flow. Using a pump, the liquid runs first through the stage's heat sink (2) and then through the objective's heat sink (26) to then returns to the pump forming a closed loop.
- **Fig. 20**: shows the connections of the inventive stage (100) and the objective heater (40) to a PID controller with 2 channels. In this example, the stage (100) is equipped with a type K thermocouple, the heat sink (2) with an NTC thermocouple, the objective heater (40) with a type K thermocouple and the heat sink (26) with an NTC thermocouple.
- **Fig. 21**: Temperature profile of the sample chamber at (**A**) 10 °C and (**B**) 30 °C. The chamber was left open on the top. There was no contact with the objective.
- **Fig. 22**: Temperature profile along the width of the sample chamber with a target temperature of 10°C.
- **Fig. 23**: Temperature profile along the width of the sample chamber with a target temperature of 30°C.
- **Fig. 24**: Temperature profile along the height of the sample chamber with a target temperature of 10°C.
- **Fig. 25**: Temperature profile along the height of the sample chamber with a target temperature of 30°C.
- **Fig. 26**: Temperature profile of the sample chamber at (**A**) 10 °C and (**B**) 30 °C. The chamber was closed on the top with a sapphire coverslip. There was no contact with the objective.
- **Fig. 27**: Temperature profile along the width of the sample chamber with a target temperature of 10°C.
- **Fig. 28**: Temperature profile along the width of the sample chamber with a target temperature of 30°C.
- **Fig. 29**: Temperature profile along the height of the sample chamber with a target temperature of 10°C.
- **Fig. 30**: Temperature profile along the height of the sample chamber with a target temperature of 30°C.
- **Fig. 31**: Temperature profile of the sample chamber at (**A**) 10 °C and (**B**) 30 °C. The chamber was closed on the top with a sapphire coverslip. There was contact with the objective at 25°C.
- **Fig. 32**: Temperature profile along the width of the sample chamber with a target temperature of 10°C.
- **Fig. 33**: Temperature profile along the width of the sample chamber with a target temperature of 30°C.
- **Fig. 34**: Temperature profile along the height of the sample chamber with a target temperature of 10°C.
- **Fig. 35**: Temperature profile along the height of the sample chamber with a target temperature of 30°C.
- **Fig. 36**: Temperature profile of the sample chamber at (**A**) 10 °C and (**B**) 30 °C. The chamber was left open on the top. There was contact with the objective that is cooled or heated to the same target temperature.
- **Fig. 37**: Temperature profile along the width of the sample chamber with a target temperature of 10°C.
- **Fig. 38**: Temperature profile along the width of the sample chamber with a target temperature of 30°C.
- **Fig. 39**: Temperature profile along the height of the sample chamber with a target temperature of 10°C.
- **Fig. 40**: Temperature profile along the height of the sample chamber with a target temperature of 30 °C.
- **Fig. 41**: shows a calibration curve obtained from an exemplary embodiment of the inventive microscope stage comprising an objective heater (40). Temperature profile along the height of the sample chamber with a target temperature of 30 °C. The temperature of the objective heater (40) was raised from 20 °C to 30 °C in 10 minutes.
- **Fig. 42**: shows the temperature distribution in the sample chamber with objective heater (40) turned off (left) and objective heater (40) turned on (right) (**A**) at 20 °C and (**B**) at 30 °C. Accuracy was determined to 0.1 °C at 20 °C and 0.5 °C at 30 °C. Precision was determined to 0.1 °C at 20 °C and 0.1 °C at 30°C.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Example 1: Temperature studies of the inventive microscope stage

The performance of the inventive microscope stage was evaluated in four different configurations.

All configurations were studied at a target temperature of 10 °C and 30 °C. Room temperature was fixed to 25 °C. To simulate the objective, a constant temperature was given to the bottom boundary of the immersion liquid. Simulations were performed using Comsol Multiphysics 6.1. The models take into consideration the cooling of the liquid by evaporation, especially important when the chamber is opened.

### 1. microscope stage with temperature control only by cooling elements (4) and an open sample chamber (3) configuration

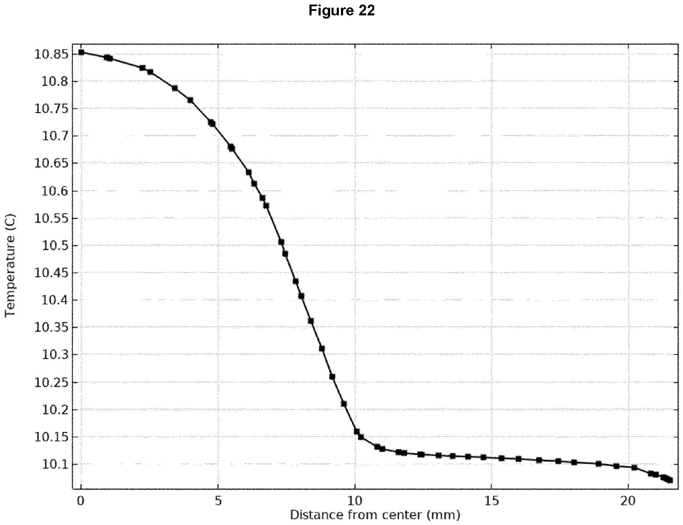

Figure 21 shows the temperature profile of the sample chamber at (A) 10 °C and (B) 30 °C. Figures 22 to 25 show the temperature profile along the width and the height of the sample chamber. The chamber was left open on the top. There was no contact with the objective.

### 2. microscope stage with temperature control only by cooling elements (4) and a sample chamber (3) closed on top with a sapphire plate.

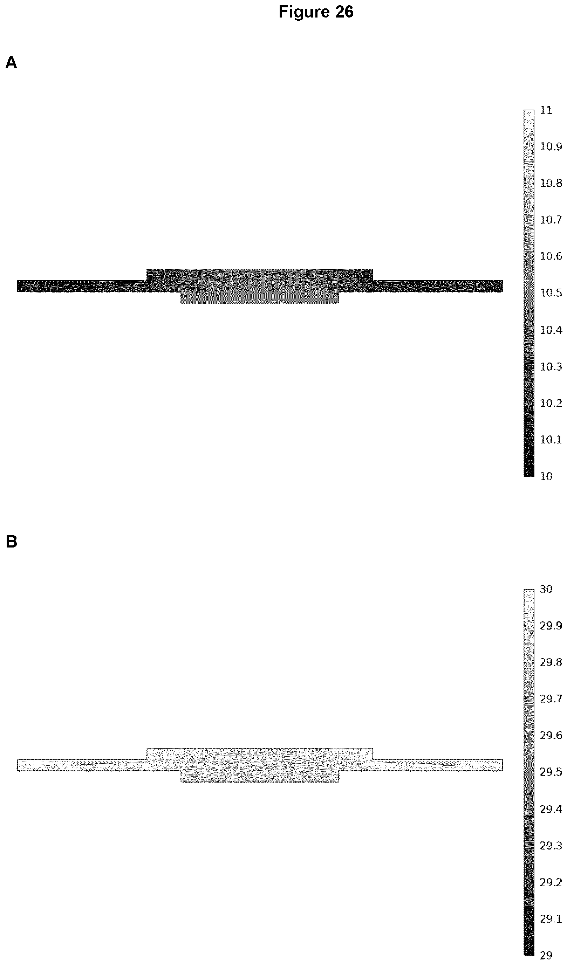

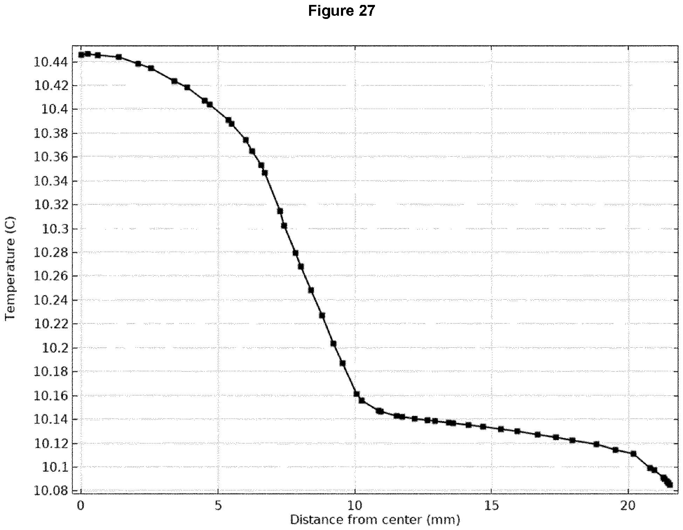

Fig. 26 shows the temperature profile of the sample chamber at (A) 10 °C and (B) 30 °C. Figures 27 to 30 show the temperature profile along the width and the height of the sample chamber. The chamber was closed on the top with a sapphire coverslip. There was no contact with the objective.

### 3. microscope stage with temperature control only by cooling elements (4) and a sample chamber (3) closed on top with a sapphire plate in contact with an objective.

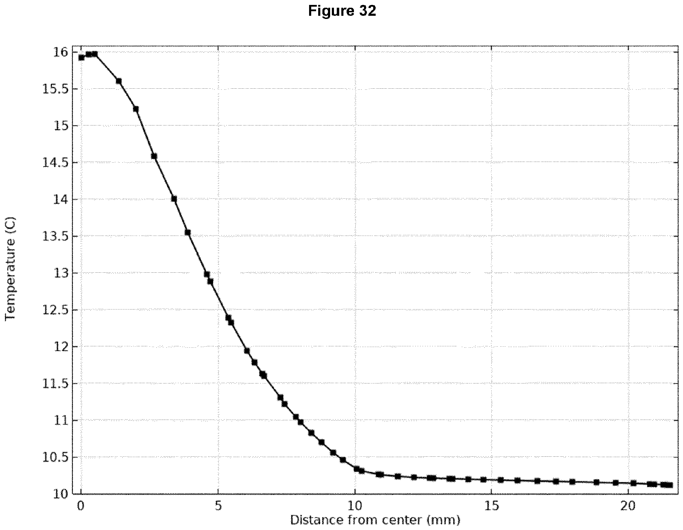

Fig. 31 shows the temperature profile of the sample chamber at (A) 10 °C and (B) 30 °C. Figures 32 to 35 show the temperature profile along the width and the height of the sample chamber. The chamber was closed on the top with a sapphire coverslip. There was contact with the objective at 25 °C.

### 4. microscope stage with temperature control by cooling elements (4) and optional cooling element (25) to control the temperature of the objective, and a sample chamber (3) closed on top with a sapphire plate in contact with an objective.

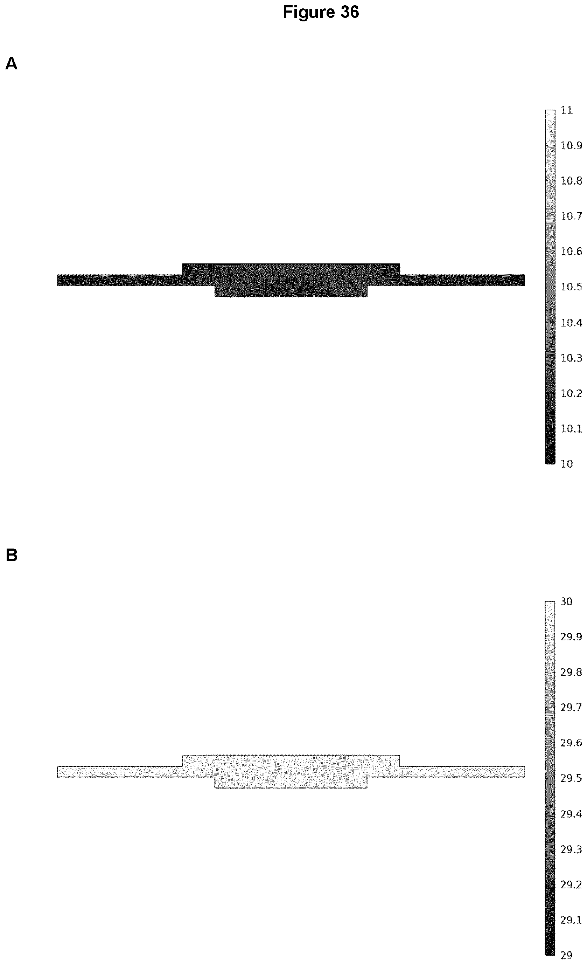

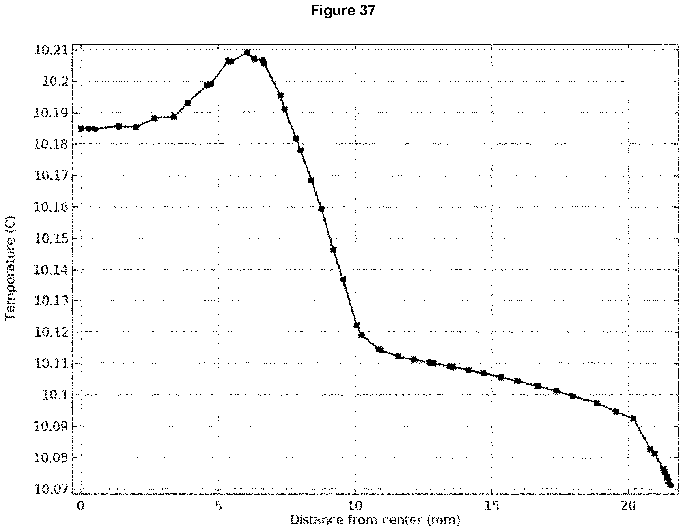

Fig. 36 shows the temperature profile of the sample chamber at (A) 10 °C and (B) 30 °C. Figures 37 to 40 show the temperature profile along the width and the height of the sample chamber. The chamber was closed on the top with a sapphire coverslip. There was contact with the objective that is cooled or heated to the same target temperature.

### Example 2: Exemplary usage of the inventive microscope stage

Before usage of the inventive microscope stage, a suitable sample should be prepared in a Petri dish first. For example, cells are incubated one day before measurement or a zebra fish embryo is mounted in an agarose dome. Before the measurement, excess liquid should be removed to only allow for enough liquid to cover the sample, for instance approximately 200 µl, in order to avoid spilling the liquid when positioning the Petri dish. The modified Petri dish is then placed in the stage and secured by using the holding pins / locking means. Further liquid may be added to the sample. To achieve a homogenous temperature gradient, the sample should be closed by overlaying a sapphire coverslip over the sample opening. The stage can now be used to change the temperature of the sample.
- Prepare sample in Petri dish
- Remove excess liquid to only be present in the glass bottom opening of the Petri dish.
- Add liquid to the chamber until full.
- Close sample opening with a sapphire coverslip.
- Start the temperature experiment by setting a target temperature on the stage

### Example 3: Exemplary usage with an immersion fluid of the inventive microscope stage equipped with an optional objective cooler

Before usage of the inventive microscope stage, a suitable sample should be prepared in a Petri dish first. For example, cells are incubated one day before measurement or a zebra fish embryo is mounted in an agarose dome. Before the measurement, excess liquid should be removed to only allow for enough liquid to cover the sample, for instance approximately 200 µl, in order to avoid spilling the liquid when positioning the Petri dish. The modified Petri dish is then placed in the stage and secured by using the holding pins / locking means. Further liquid may be added to the sample. To achieve a homogenous temperature gradient, the sample should be closed by overlaying a sapphire coverslip over the sample opening. The stage can now be used to change the temperature of the sample.
- Prepare sample in Petri dish
- Remove excess liquid to only be present in the glass bottom opening of the Petri dish.
- Add liquid to the chamber until full.
- Close sample opening with a sapphire coverslip.
- Start the temperature experiment by setting a target temperature on the stage
- Regulate the temperature of the objective with the optional objective cooler.

### Reference signs

- 1: heat transfer plate
- 2: heat sink
- 3: modified Petri dish
- 4: cooling elements for controlling the temperature of the Petri dish
- 5: body of the heat transfer plate
- 6: opening in the cylinder (131) for sample visualization
- 7: locking means
- 8: channel for inserting a thermocouple
- 9: coverslip
- 10: insert
- 11a: thin cut outs in the heat transfer plate
- 11b: thin cut outs in the heat sink
- 12: top plate of the heat sink
- 13: lid of the heat sink
- 14: circular opening in the top plate (12)
- 15: circular opening in the lid (13)
- 16: holes for positioning of the microscope stage
- 17: cooling liquid channel
- 18: cooling liquid inlet and outlet
- 19: opening for positioning a thermocouple
- 20: Petri dish body
- 21: cut outs of the Petri dish body
- 22: coverslip
- 23: opening in the Petri dish body
- 24: heat conductive element
- 25: cooling element
- 26: liquid cooling heat sink
- 27: body of the heat conductive element
- 28: holding means
- 28a: elastic band
- 29: thin cut out for positioning the cooling element
- 30: heat sink lid
- 31: heat sink body
- 32: channel for liquid cooling
- 33: inlet and outlet for cooling liquid
- 34: hole for positioning a thermocouple for temperature measurement of the heat sink (26)
- 35: microscope objective
- 36: spacer
- 40: objective heater
- 100: microscope stage
- 110: cooling device
- 112: Peltier element
- 113: Peltier element
- 114: PID controller
- 120: support
- 122: circularly shaped opening
- 131: cylinder open on both ends
- 136: live cell sample
- 141: thermocouple
- 142: thermocouple
- 143: thermocouple
- 150: computer
- 1000: microscope

## Claims

1. A microscope stage (100) for temperature-controlled live cell imaging comprising:
(a) a cooling device (110) comprising at least one cooling element (4);
(b) a heat transfer plate (1) comprising a cylinder open on both ends (131), the cylinder (131) extending from the plate (1) and being adapted for receiving opposite to the plate (1) a Petri dish (3) containing a live cell sample (136) in thermal communication; wherein the cylinder (131) comprises at least one opening (6) in its wall and a channel configured to accommodate a thermocouple (141) at the end of the cylinder opposite to the plate (1); and
(c) a heat sink (2) configured as support (120) comprising a circularly shaped opening (122),
wherein the cooling device (110) is mounted on the support (120) to enable the heat sink (2) to dissipate heat produced by the at least one cooling element (4), and
wherein the heat transfer plate (1) is arranged on the support (120) in thermal communication with the at least one cooling element (4), such that the cylinder (131) of the heat transfer plate (1) protrudes into the circularly shaped opening (122) of the support (120) to allow illumination of the live cell sample (136).

2. The microscope stage (100) according to claim 1, wherein the opening of the cylinder (131) opposite to the plate is covered from inside the cylinder with a transparent coverslip (9) made of sapphire or glass.

3. The microscope stage (100) according to claim 1 or 2, wherein an insert (10) made of sapphire is placed between the opening of the cylinder (131) opposite to the plate (1) and the Petri dish (3) to reduce the sample volume.

4. The microscope stage (100) according to any one of claims 1 to 3, wherein the cylinder (131) comprises a pin for holding the Petri dish (3) in position.

5. The microscope stage (100) according to any one of claims 1 to 4, wherein the heat sink (2) comprises a top plate (12) and a lid (13) having a circular opening (15), the top plate (12) comprising a circular opening (14), a cooling liquid channel (17) connected to an inlet and outlet (18) of the top plate (12), and an opening (19) configured to accommodate a thermocouple (142).

6. The microscope stage (100) according to any one of the claims 1 to 5, wherein the stage further comprises an objective heater (40) comprising a heat conductive element (24) adapted to encompass the objective with an elastic band (28a), a cooling element (25) for controlling the temperature of the objective, a liquid cooling heat sink (26) for dissipating the heat generated by the cooling element (25).

7. The microscope stage (100) according to claim 6, wherein the liquid cooling heat sink (26) comprises a lid (30) and a body (31), wherein the body (31) comprises a channel (32) for liquid cooling connected to an inlet and outlet (33), and an opening (34) configured to accommodate a thermocouple (143) for measuring the temperature of the liquid cooling heat sink (26).

8. The microscope stage (100) according claim 6 or 7, wherein the stage (100) further comprises a spacer (36) for thermal isolation positioned between the objective and the microscope body.

9. The microscope stage (100) according to any one of claims 1 to 8, wherein the at least one cooling element (4) are two Peltier elements (112,113).

10. The microscope stage (100) according to claim 9, wherein the two Peltier elements (112,113) are controlled by a PID controller (114) connected to a computer (150).

11. The microscope stage (100) according to any one of the claims 1 to 10, wherein the heat transfer plate (1) is made of a heat conductive material, preferably aluminium.

12. The microscope stage (100) according to any one of the claims 1 to 11, wherein the heat sink (2) is made of a heat conductive material, preferably aluminium.

13. A microscope (1000) comprising a microscope stage (100) according to any one of claims 1 to 12.
